# EUROPÄISCHE PATENTSCHRIFT

(11) **EP 1 732 877 B1**
(45) Veröffentlichungstag und Bekanntmachung des Hinweises auf die Patenterteilung: **17.06.2015**
(21) Anmeldenummer: 05728075.2
(22) Anmeldetag: 26.03.2005
(51) Int. Cl.: C07C 67/08, C07C 69/33, A61K 8/06, A61K 8/37, A61Q 19/00, C11C 3/00

(54) **SENSORISCHE WACHSE FÜR KOSMETISCHE UND/ODER PHARMAZEUTISCHE FORMULIERUNGEN**
SENSORY WAX FOR COSMETIC AND/OR PHARMACEUTICAL FORMULATIONS
CIRES SENSORIELLES DESTINEES A DES FORMULATIONS COSMETIQUES ET/OU PHARMACEUTIQUES

(30) Priorität: 05.04.2004 DE 102004017222
(43) Veröffentlichungstag der Anmeldung: 20.12.2006
(73) Patentinhaber: Cognis IP Management GmbH, 40589 Düsseldorf (DE)
(72) Erfinder: ISSBERNER, Ulrich, Ambler, PA 19002 (US); MITCHELL, Catherine, 40223 Düsseldorf (DE); ANSMANN, Achim, 40699 Erkrath (DE); BRÜNING, Stefan, Philadelphia, PA 19118 (US); GONDEK, Helga, 40589 Düsseldorf (DE)
(74) Vertreter: Reinhardt, Jürgen
(86) Internationale Anmeldenummer: PCT/EP2005/003234
(87) Internationale Veröffentlichungsnummer: WO 2005/097044

(56) Entgegenhaltungen:
- EP-A- 0 163 806
- EP-A- 0 179 416
- EP-A- 1 216 685
- EP-A- 1 290 999
- WO-A-99/62468
- WO-A-03/028690
- US-A- 3 976 789
- US-A- 4 113 635
- US-A- 4 290 337
- US-A- 4 332 702
- US-A- 5 741 919
- BREUSCH, F.L. ET AL.: "Darstellung der di-, tri- und tetra-homologen Reihen der Methan-methylol-fettsäureester (XIV. Mitteil. über isomere und homologe Reihen)" CHEM. BER., Bd. 88, Nr. 10, 1955, Seiten 1511-1519, XP009051613

## Beschreibung

### Gebiet der Erfindung

Die Erfindung betrifft wachsartige Ester von Pentaerythrit und ein Verfahren zu deren Herstellung. Die Erfindung betrifft auch Zusammensetzungen auf Basis dieser Ester und deren Verwendung in kosmetischen und/oder pharmazeutischen Zusammensetzungen bzw. als Konsistenzgeber.

### Stand der Technik

Formulierungen auf Wachsbasis sind dem Fachmann seit langem bekannt. Diese werden u.a. für kosmetische und pharmazeutische Formulierungen verwendet, wie z.B. Suppositorien, verschiedene kosmetische Zusammensetzungen in Stiftform oder in Cremes und Lotionen, zur Beschichtung von Papieren und Textilien, etc. Dem Fachmann stehen hierfür zahlreiche wachsartigen Substanzen und Substanzgemische zur Verfügung. Hierzu gehören beispielsweise Glyceride und Fettalkohole, die sich entscheidend auf die Sensorik der Endformulierungen auswirken. So hinterlassen Glyceride ein oft öliges/fettiges Hautgefühl. Fettalkohole führen zu einer stärkeren weißen Rückstandsbildung, die von den Anwendern als sehr nachteilig empfunden wird. Um dieser weißen Rückstandsbildung entgegenzuwirken, werden häufig Silikonöle zugesetzt. Silikonöle werden außerdem eingesetzt, um die Klebrigkeit der Formulierungen zu reduzieren und um das Glätte- und Samtigkeitgefühl bei Anwendung auf der Haut zu verbessern. Aus toxikologischer und ökologischer Sicht haben einige Silikonöle jedoch Nachteile. Deswegen wird seit geraumer Zeit nach Ersatzstoffen für Silikonöle gesucht.

Aufgabe der vorliegenden Erfindung war es, wachsartige Substanzen und Zusammensetzungen auf Basis dieser Substanzen zur Verfügung zu stellen, die gegenüber den üblicherweise verwendeten Wachsen zusätzliche sensorische Vorteile aufweisen, die normalerweise durch Silikonöle erreicht werden. Derartige Wachse erlauben es, Kosmetika ohne Silikonöle zu formulieren, ohne dabei auf das typische sensorische Profil, das Silikonölen eigen ist, zu verzichten.

Überraschenderweise wurde nun gefunden, dass sich diese Eigenschaften durch spezielle Wachse auf Basis von Pentaerythrit oder dessen Oligomeren erreichen lassen, die nicht aus tierischen, sondern aus pflanzlichen Rohstoffquellen hergestellt werden.

Pentaerythritester sind seit langem auf dem Markt und werden u.a. als Emulgatoren in der Lebensmittelindustrie eingesetzt. Diese stammen aus tierischen Rohstoffquellen. Nur beispielhaft ist hier ein Produkt der Cognis Deutschland GmbH & Co. KG zu nennen, das unter der Bezeichnung Loxiol® P 728 im Handel ist. Es weist ca. 2% Gew.-% C17-Fettsäuren auf und hat eine C16/C18-Verteilung von ca. 30:70 und entspricht damit anderen marktüblichen Produkten. Derartige Wachsester sind bezüglich Stabilität, Viskosität, makroskopischen/mikroskopischen Erscheinungsbild und bezüglich des sensorischen Profils der damit hergestellten kosmetischen Zusammensetzungen jedoch nicht völlig zufriedenstellend.

XP 009051613 beschreibt Einzelverbindungen, d.h. Ester von Pentaerythrit mit verschiedenen Fettsäuren. Estergemische sind nicht offenbart, ebenso wenig kosmetische Zubereitungen, welche solche Estergemische enthalten.

US 4,290,337 beschreibt Zubereitungen zum Beschichten von Muttern. Es werden keine kosmetischen Zubereitungen beschrieben.

US 4,113,635 beschreibt rostfreie Schmiermittel für die Beschichtung von Metallen. Diese enthalten Pentaerythritester-Gemische. Die Beispiele beschreiben solche Schmiermittelzubereitungen, welche Pentaerythritester von "beef tallow", also Rindertalg enthalten. Rindertalg enthält mehr als 0,3 Gew.-% C17 Fettsäuren.

US 4,332,702 beschreibt Polyvinylchlorid-Rohrverbindungen, welche durch Aufschmelzen von Zubereitungen erhalten werden, die Pentaerythritester-Gemische enthalten. Es werden weder kosmetische Zubereitungen noch Zubereitungen, welche ein Pentaerythritester Gemisch enthalten, welches fast C17- Fettsäureacylgruppen frei ist, beschrieben.

EP 179416 A beschreibt kosmetische Zubereitungen, welche einzelne Ester von Pentaerythrit enthalten.

WO 99/62468 beschreibt kosmetische Zubereitungen, welche flüssige Pentaerythrit-Tetraester enthalten.

EP 163806 A beschreibt Gemische von partiell veresterten Oligomeren von Pentaerythrit.

US 3,976,789 beschreibt kosmetische Zubereitungen, welche Tri- oder Tetraester von Pentaerythrit mit 2- Ethylhexansäure, 2-Heptylundecansäure oder 2-Hexyldecansäure enthalten. Es werden keine kosmetischen Zubereitungen beschrieben, welche Gemische_von Estern enthalten.

US 5,741,919 beschreibt Ester von "meadow foam oil" und ihre Verwendung in kosmetischen Zubereitungen.

EP 1290999 A beschreibt kosmetische Zubereitungen, welche Pentaerythrityltetraisostearat enthält. Kosmetische Zubereitungen, welche Gemische von Pentaerythritestern enthalten, sind nicht beschrieben.

EP 1216685 A beschreibt ebenfalls kosmetische Zubereitungen, welche Pentaerythrit-Tetraoctanoate oder Pentaerythrit- Tetrapelargonat enthalten. Kosmetische Zubereitungen, welche Gemische von Pentaerythritestern enthalten, sind nicht beschrieben.

WO 03/028690 beschreibt kosmetische Zubereitungen, welche Pentaerythrityl-Tetraisostearat enthält. Kosmetische Zubereitungen, welche Gemische von Pentaerythritestern enthalten, sind nicht beschrieben.

### Beschreibung der Erfindung

Gegenstand der Erfindung sind daher Ester von C6-C22-Fettsäuren des Pentaerythrits, die weniger als 0.3 Gew.-% C17-Fettsäureacylgruppen enthalten und einen Schmelzpunkt von wenigstens 30° C aufweisen und 12 - 19 Gew.-% Monoester, (b) 25 - 35 Gew.-% Diester, (c) 30 - 40 Gew.-% Triester und (d) 6 -11 Gew.-% Tetraester enthalten Überraschenderweise wurde gefunden, dass diese Ester in kosmetischen Zusammensetzungen ein sehr gutes sensorisches Eigenschaftsprofil aufweisen.

Die Ester können eine einzige Art von Fettsäure-Acylgruppen aufweisen oder ein Gemisch verschiedener Fettsäure-Acylgruppen, die Fettsäuren können verzweigt oder unverzweigt und/oder gesättigt oder ungesättigt sein. Vorzugsweise werden für die Veresterung Fettsäuren mit einem hohen Gehalt an gesättigten unverzweigten Fettsäuren eingesetzt, insbesondere solche, die aus pflanzlichen Rohstoffquellen stammen. Erfindungsgemäß bevorzugt sind C14-C24-Fettsäuren, insbesondere C14-C20-Fettsäuren.

Eine weitere bevorzugte Ausführungsform der Ester ist dadurch gekennzeichnet, dass man für die Veresterung ein Fettsäuregemisch enthaltend 40 - 50 Gew.-% C16-Fettsäure und 45 - 55 Gew.-% C18-Fettsäure einsetzt. Die Restmengen des Fettsäuregemisches sind kürzerkettige (≤ C14) und längerkettige (> C18) Fettsäuren. Erfindungsgemäß bevorzugt geeignet ist ein Ester des Pentaerythrits, der durch Umsetzung von Pentaerythrit mit einem Fettsäuregemisch enthaltend 42 - 48 Gew.-% C16-Fettsäure und 50 - 56 Gew.-% C18-Fettsäure (restliche Mengen: ≤ C14-Fettsäuren und > C18-Fettsäuren) erhalten wird.. Üblicherweise werden pro Mol Pentaerythrit 1.8 - 2.2 Mol des Fettsäuregemisches, vorzugsweise 1.9 - 2.1 Mol, für die Veresterung eingesetzt.

Gegenstand der Erfindung ist auch ein Verfahren zur Herstellung von C16/C18-Fettsäure-Pentaerythritestern, wobei man pro 1 Mol Pentaerythrit 1.8 - 2.2 Mol, vorzugsweise 1.9 - 2.1 Mol, eines Fettsäuregemisches enthaltend 40 - 50 Gew.-% C16-Fettsäure und 45 - 55 Gew.-% C18-Fettsäure oder ein Rohstoffgemisch mit entsprechender Fettsäureverteilung einsetzt und (a) die Veresterung bei Temperaturen im Bereich von 180°C bis 250°C unter Schutzgasatmosphäre, und ohne Lösungsmittel durchführt, (b) das entstehende Wasser abdestilliert, (c) das erhaltene Reaktionsgemisch im Vakuum solange nachrührt, bis eine Säurezahl von <1 und eine OH-Zahl von 145 - 158 erreicht ist, (d) nicht abreagiertes Pentaerythrit abfltriert und (e) ggf. eine Nachbehandlung mit Wasserstoffperoxid durchführt. Dem Fachmann sind die Methoden zur Kontrolle und Einstellung der Säurezahl und der OH-Zahl hinlänglich bekannt, so dass an dieser Stelle nicht näher darauf eingegangen werden muss.

Gegenstand der Erfindung ist auch die Verwendung der erfindungsgemäßen Ester als Konsistenzgeber.

### Gewerbliche Anwendbarkeit

Kosmetische und pharmazeutische Zusammensetzungen, welche die erfindungsgemäßen Ester als Bestandteile enthalten, sind im Vergleich zu ähnlichen Zusammensetzungen des Standes der Technik bezüglich Stabilität, Viskosität, makroskopischen/mikroskopischen Erscheinungsbild und/oder bezüglich des sensorischen Profils überlegen. Sie sind leicht verteilbar, ziehen gut auf die Haut auf, hinterlassen ein relativ geringes öliges, klebriges oder fettendes, sondern vielmehr samtiges und glattes Hautgefühl. Ein weiterer Gegenstand der Anmeldung sind daher kosmetische und/oder pharmazeutische Zusammensetzungen, welche die erfindungsgemäßen Ester enthalten, in einer Menge von 0,1 - 20 Gew.-% bezogen auf die Gesamtzusammensetzung. Die erfindungsgemäßen Zusammensetzungen enthalten Ester von C6-C22-Fettsäuren des Pentaerythrits, mit einem Anteil von (a) 5 - 35 Gew.-% Monoester, (b) 20 - 50 Gew.-% Diester und (c) 25 - 50 Gew.-% Triester, und ggf. Tetraester. Besonders bevorzugt ist ein Gehalt an (a) 10 - 25 Gew.-% Monoester, (b) 25 - 40 Gew.-% Diester und (c) 30 - 45 Gew.-% Triester, und ggf. Tetraester und ganz besonders bevorzugt (a) 12 - 19 Gew.-% Monoester, (b) 25 - 35 Gew.-% Diester, (c) 30 - 40 Gew.-% Triester und (d) 6 -11 Gew.-% Tetraester. Bei der Umsetzung von Pentaerythrit mit einem Fettsäuregemisch enthaltend 42 - 48 Gew.-% C16-Fettsäure und 50 - 56 Gew.-% C18-Fettsäure (restliche Mengen: ≤ C14-Fettsäuren und > C18-Fettsäuren) erhält man folgende Esterverteilung: 12 - 19 Gew.-% Monoester, (b) 25 - 35 Gew.-% Diester, (c) 30 - 40 Gew.-% Triester und (d) 6 - 11 Gew.-% Tetraester. Diese Zusammensetzungen haben ein besonders gutes Eigenschaftsprofil hinsichtlich Stabilität, Viskosität, makroskopischen/ mikroskopischen Erscheinungsbild und bezüglich Sensorik. Eine bevorzugte Ausführungsform der erfindungsgemäßen Zusammensetzung ist frei von Siliconölen. Gegenstand der Erfindung ist auch die Verwendung der erfindungsgemäßen Zusammensetzung in kosmetischen und/ oder pharmazeutischen Zusammensetzungen bzw. als Konsistenzgeber.

### Weitere Wachskomponenten

Eine weitere bevorzugte Ausführungsform der Erfindung enthält wenigstens eine weitere Wachskomponente. Unter dem Begriff Wachs werden üblicherweise alle natürlichen oder künstlich gewonnenen Stoffe und Stoffgemische mit folgenden Eigenschaften verstanden: sie sind von fester bis brüchig harter Konsistenz, grob bis feinkristallin, durchscheinend bis trüb und schmelzen oberhalb von 30°C ohne Zersetzung. Sie sind schon wenig oberhalb des Schmelzpunktes niedrigviskos und nicht fadenziehend und zeigen eine stark temperaturabhängige Konsistenz und Löslichkeit. Erfindungsgemäß einsetzbar ist eine Wachskomponente oder ein Gemisch von Wachskomponenten, die bei 30°C oder darüber schmelzen. Sie sind in den erfindungsgemäßen Zusammensetzungen in einer Gesamtmenge von 0,1 - 15 Gew.-% enthalten. In einer bevorzugten Ausführungsform der Erfindung liegt der Gehalt der zusätzlichen Wachskomponente(n) bei 0,1 - 10 Gew.-% bezogen auf die Gesamtzusammensetzung, vorzugsweise 0,5 - 5 Gew.-% und insbesondere 1 - 5 Gew.-% bezogen auf die Gesamtzusammensetzung.

Als Wachse können erfindungsgemäß auch Fette und fettähnliche Substanzen mit wachsartiger Konsistenz eingesetzt werden, solange sie den geforderten Schmelzpunkt haben. Hierzu gehören u.a. Fette (Triglyceride), Mono- und Diglyceride, natürliche und synthetische Wachse, Fett- und Wachsalkohole, Fettsäuren, Ester von Fettalkoholen und Fettsäuren sowie Fettsäureamide oder beliebige Gemische dieser Substanzen.

Unter Fetten versteht man Triacylglycerine, also die Dreifachester von Fettsäuren mit Glycerin. Bevorzugt enthalten sie gesättigte, unverzweigte und unsubstituierte Fettsäurereste. Hierbei kann es sich auch um Mischester, also um Dreifachester aus Glycerin mit verschiedenen Fettsäuren handeln. Erfindungsgemäß einsetzbar und als Konsistenzgeber besonders gut geeignet sind sogenannte gehärtete Fette und Öle, die durch Partialhydrierung gewonnen werden. Pflanzliche gehärtete Fette und Öle sind bevorzugt, z. B. gehärtetes Rizinusöl, Erdnußöl, Sojaöl, Rapsöl, Rübsamenöl, Baumwollsaatöl, Sojaöl, Sonnenblumenöl, Palmöl, Palmkernöl, Leinöl, Mandelöl, Maisöl, Olivenöl, Sesamöl, Kakaobutter und Kokosfett.

Geeignet sind u.a. die Dreifachester von Glycerin mit C₁₂-C₆₀-Fettsäuren und insbesondere C₁₂-C₃₆-Fettsäuren. Hierzu zählt gehärtetes Rizinusöl, ein Dreifachester aus Glycerin und einer Hydroxystearinsäure, der beispielsweise unter der Bezeichnung Cutina^{®} HR im Handel ist. Ebenso geeignet sind Glycerintristearat, Glycerintribehenat (z. B. Syncrowax^{®} HRC), Glycerintripalmitat oder die unter der Bezeichnung Syncrowax^{®} HGLC bekannten Triglycerid-Gemische, mit der Vorgabe, daß der Schmelzpunkt der Wachskomponente bzw. des Gemisches bei 30 °C oder darüber liegt.

Als Wachskomponenten sind erfindungsgemäß insbesondere Mono- und Diglyceride bzw. Mischungen dieser Partialglyceride einsetzbar. Zu den erfindungsgemäß einsetzbaren Glyceridgemischen zählen die von der Cognis Deutschland GmbH & Co. KG vermarkteten Produkte Novata^{®} AB und Novata^{®} B (Gemisch aus C₁₂-C₁₈-Mono-, Di- und Triglyceriden) sowie Cutina^{®} MD oder Cutina^{®} GMS (Glycerylstearat). Erfindungsgemäß besonders gut geeignet sind C12-C24-Partialglyceride als weitere Wachskomponente.

Mischester sowie Mischungen aus Mono-, Di- und Triglyceriden sind erfindungsgemäß bevorzugt geeignet, da sie eine geringere Neigung zur Kristallisation zeigen und somit die Performance der erfindungsgemäßen Zusammensetzung verbessern. Sie sind darüber hinaus wesentlich besser mit Ölen verschiedenster Polarität kompatibel.

Zu den erfindungsgemäß einsetzbaren **Fettalkoholen** zählen die C₁₂-C₅₀-Fettalkohole. Erfindungsgemäß bevorzugt geeignet als weitere Wachskomponente sind C₁₂-C₂₄-Fettalkohole, die auch in Kombination mit den C12-C24 Partialglyceriden einsetzbar sind. Die Fettalkohole können aus natürlichen Fetten, Ölen und Wachsen gewonnen werden, wie beispielsweise Myristylalkohol, 1-Pentadecanol, Cetylalkohol, 1-Heptadecanol, Stearylalkohol, 1-Nonadecanol, Arachidylalkohol, 1-Heneicosanol, Behenylalkohol, Brassidylalkohol, Lignocerylalkohol, Cerylalkohol oder Myricylalkohol. Erfindungsgemäß bevorzugt sind gesättigte unverzweigte Fettalkohole. Aber auch ungesättigte, verzweigte oder unverzweigte Fettalkohole können erfindungsgemäß als Wachskomponente verwendet werden, solange sie den geforderten Schmelzpunkt aufweisen. Erfindungsgemäß einsetzbar sind auch Fettalkoholschnitte, wie sie bei der Reduktion natürlich vorkommender Fette und Öle wie z. B. Rindertalg, Erdnußöl, Rüböl, Baumwollsaatöl, Sojaöl, Sonnenblumenöl, Palmkernöl, Leinöl, Rizinusöl, Maisöl, Rapsöl, Sesamöl, Kakaobutter und Kokosfett anfallen. Es können aber auch synthetische Alkohole, z. B. die linearen, geradzahligen Fettalkohole der Ziegler-Synthese (Alfole^{®}) oder die teilweise verzweigten Alkohole aus der Oxosynthese (Dobanole^{®}) verwendet werden. Erfindungsgemäß besonders bevorzugt geeignet sind C₁₄-C₂₂-Fettalkohole, die beispielsweise von der Cognis Deutschland GmbH unter der Bezeichnung Lanette^{®} 16 (C₁₆-Alkohol), Lanette^{®} 14 (C₁₄-Alkohol), Lanette^{®} O (C₁₆/C₁₈-Alkohol) und Lanette^{®} 22 (C₁₈/C₂₂-Alkohol) vermarktet werden. Fettalkohole verleihen den Zusammensetzungen ein trockeneres Hautgefühl als Triglyceride und sind daher gegenüber letzteren bevorzugt.

Als Wachskomponenten können auch C₁₄-C₄₀-**Fettsäuren** oder deren Gemische eingesetzt werden. Hierzu gehören beispielsweise Myristin-, Pentadecan-, Palmitin-, Margarin-, Stearin-, Nonadecan-, Arachin-, Behen-, Lignocerin-, Cerotin-, Melissin-, Eruca- und Elaeostearinsäure sowie substituierte Fettsäuren, wie z. B. 12-Hydroxystearinsäure, und die Amide oder Monoethanolamide der Fettsäuren, wobei diese Aufzählung beispielhaften und keinen beschränkenden Charakter hat.

Erfindungsgemäß verwendbar sind beispielsweise **natürliche pflanzliche Wachse,** wie Candelillawachs, Carnaubawachs, Japanwachs, Espartograswachs, Korkwachs, Guarumawachs, Reiskeimölwachs, Zuckerrohrwachs, Ouricurywachs, Montanwachs, Sonnenblumenwachs, Fruchtwachse wie Orangenwachse, Zitronenwachse, Grapefruitwachs, Lorbeerwachs (=Bayberrywax) und **tierische Wachse,** wie z. B. Bienenwachs, Schellackwachs, Walrat, Wollwachs und Bürzelfett. Im Sinne der Erfindung kann es vorteilhaft sein, hydrierte oder gehärtete Wachse einzusetzen. Zu den erfindungsgemäß verwendbaren natürlichen Wachsen zählen auch die **Mineralwachse,** wie z. B. Ceresin und Ozokerit oder die petrochemischen Wachse, wie z. B. Petrolatum, Paraffinwachse und Mikrowachse. Als Wachskomponente sind auch **chemisch modifizierte Wachse,** insbesondere die Hartwachse, wie z. B. Montanesterwachse, Sasolwachse und hydrierte Jojobawachse einsetzbar. Zu den **synthetischen Wachsen,** die erfindungsgemäß einsetzbar sind, zählen beispielsweise wachsartige Polyalkylenwachse und Polyethylen-glycolwachse. Pflanzliche Wachse sind erfindungsgemäß bevorzugt.

Die weitere Wachskomponente kann ebenso gewählt werden aus der Gruppe der **Wachsester** aus gesättigten und/oder ungesättigten, verzweigten und/oder unverzweigten Alkancarbonsäuren und gesättigten und/oder ungesättigten, verzweigten und/oder unverzweigten Alkoholen, aus der Gruppe der Ester aus aromatischen Carbonsäuren, Dicarbonsäuren, Tricarbonsäuren bzw. Hydroxycarbonsäuren (z. B. 12-Hydroxystearinsäure) und gesättigten und/oder ungesättigten, verzweigten und/oder unverzweigten Alkoholen, sowie ferner aus der Gruppe der Lactide langkettiger Hydroxycarbonsäuren. Beispiel solcher Ester sind die C₁₆-C₄₀-Alkylstearate, C₂₀-C₄₀-Alkylstearate (z. B. Kesterwachs^{®} K82H), C₂₀-C₄₀-Dialkylester von Dimersäuren, C₁₈-C₃₈-Alkylhydroxystearoylstearate oder C₂₀-C₄₀-Alkylerucate. Ferner sind C₃₀-C₅₀-Alkylbienenwachs, Tristearylcitrat, Triisostearylcitrat, Stearylheptanoat, Stearyloctanoat, Trilaurylcitrat, Ethylenglycoldipalmitat, Ethylenglycoldistearat, Ethylenglykoldi(12-hydroxystearat), Stearylstearat, Palmitylstearat, Stearylbehenat, Cetearylbehenat und Behenylbehenat einsetzbar.

### Ölkörper

Gemäß einer weiteren bevorzugten Ausführungsform enthält die erfindungsgemäße Zusammensetzung zusätzlich wenigstens eine bei 25 °C flüssige Ölkomponente oder ein beliebiges Gemisch solcher Ölkomponenten. Die Ölkomponente(n) ist vorzugsweise in einer Menge von 1 - 25 Gew.-%, insbesondere 1 - 15 Gew.-% und ganz bevorzugt 5 - 15 Gew.-% bezogen auf die Gesamtzusammensetzung enthalten.

Als Ölkörper sind beispielsweise die nachstehend genannten Verbindungsklassen geeignet, soweit diese bei 25 °C flüssig sind. Hierzu zählen u.a. Guerbetalkohole auf Basis von Fettalkoholen mit 6 bis 18, vorzugsweise 8 bis 10 Kohlenstoffatomen in Frage, Ester von linearen oder verzweigten, gesättigten oder ungesättigten C₆-C₂₂-Fettsäuren mit linearen oder verzweigten, gesättigten oder ungesättigten C₆-C₂₂-Fettalkoholen, insbesondere 2-Ethylhexanol. Beispielhaft seien genannt Hexyllaurat, Myristylisostearat, Myristyloleat, Cetylisostearat, Cetyloleat, Stearylisostearat, Stearyloleat, Isostearylmyristat, Isostearylpalmitat, Isostearylstearat, Isostearylisostearat, Isostearyloleat, Oleylmyristat, Oleylisostearat, Oleyloleat, Oleylerucat, Erucylisostearat, Erucyloleat, Cococaprylat/caprat. Weitere geeignete Ester sind z.B. Ester von C₁₈-C₃₈-Alkylhydroxycarbonsäuren mit linearen oder verzweigten, gesättigten oder ungesättigten C₆-C₂₂-Fettalkoholen, Ester von linearen und/oder verzweigten, gesättigten oder ungesättigten Fettsäuren mit mehrwertigen Alkoholen (wie z. B. Propylenglycol, Dimerdiol oder Trimertriol) und/oder Guerbetalkoholen, Triglyceride oder Triglyceridmischungen, flüssige Mono-/Di-/Triglyceridmischungen, Ester von C₆-C₂₂-Fettalkoholen und/oder Guerbetalkoholen mit aromatischen Carbonsäuren, insbesondere Benzoesäure, Ester von C₂-C₁₂-Dicarbonsäuren mit linearen oder verzweigten, gesättigten oder ungesättigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane, lineare Dialkylcarbonate, Guerbetcarbonate auf Basis von Fettalkoholen mit 6 bis 18, vorzugsweise 8 bis 10 C Atomen, Ester der Benzoesäure mit linearen und/oder verzweigten C₆-C₂₂-Alkoholen (z. B. Finsolv® TN), lineare oder verzweigte, symmetrische oder unsymmetrische Dialkylether mit 6 bis 22 Kohlenstoffatomen pro Alkylgruppe, wie z. B. Di-n-octyl Ether (Cetiol® OE) oder Ringöffnungsprodukte von epoxidierten Fettsäureestern mit Polyolen, Kohlenwasserstoffe wie Paraffin- oder Mineralöle, Oligo- oder Polyalphaolefine.

Dialkylether, Dialkylcarbonate, Triglycerid-Mischungen und Ester aus C8-C24-Fettsäuren und C8-C24 Fettalkoholen bzw. ein Gemisch dieser Substanzen sind erfindungsgemäß als Ölkörper besonders gut geeignet. Die Dialkylcarbonate und Dialkylether können symmetrisch oder unsymmetrisch, verzweigt oder unverzweigt, gesättigt oder ungesättigt sein und lassen sich nach Reaktionen, die aus dem Stand der Technik hinlänglich bekannt sind, herstellen. Erfindungsgemäß bevorzugt ist es, ein Gemisch aus Ölkörpern einzusetzen, das Ester, Dialkylether und Triglyceride enthält und frei von Silikonölen ist.

Erfindungsgemäß einsetzbar sind u.a. auch Kohlenwasserstoffe, vorzugsweise mit einer Kettenlänge 8 bis 40 C-Atomen aus. Sie können verzweigt oder unverzweigt sein, gesättigt oder ungesättigt. Unter diesen sind verzweigte, gesättigte C8-C40-Alkane bevorzugt. Es können sowohl Reinsubstanzen eingesetzt werden als auch Substanzgemische. Üblicherweise handelt es sich um Substanzgemische verschiedener isomerer Verbindungen. Zusammensetzungen, die Alkane mit 10 bis 30, vorzugsweise 12 bis 20, und besonders bevorzugt 16 bis 20 Kohlenstoffatome aufweisen, sind besonders geeignet, und unter diesen ein Gemisch aus Alkanen, welches wenigstens 10 Gew.-% verzweigte Alkane bezogen auf die Gesamtmenge der Alkane enthält. Vorzugsweise handelt es sich um verzweigte, gesättigte Alkane. Besonders gut geeignet sind Gemische aus Alkanen ist, welche mehr als 1 Gew.-% 5,8-Diethyldodecan und/oder mehr als 1 Gew.-% Didecen enthalten.

### Oberflächenaktive Substanzen

Eine weitere bevorzugte Ausführungsform der erfindungsgemäßen Zusammensetzung enthält zusätzlich wenigstens einen nichtionischen grenzflächenaktiven Stoff. Dieser trägt zur Stabilisierung der erfindungsgemäßen Formulierungen bei. Der Gehalt des nichtionischen oberflächenaktiven Stoffs hängt von der Art der Formulierung ab, übersteigt aber üblicherweise 10 Gew.-% nicht. Der bevorzugte Gehalt liegt zwischen 0,5 - 10 Gew.-%, vorzugsweise 0,5 - 5 Gew.-% und insbesondere 0,5 - 3 Gew.-% bezogen auf die Gesamtzusammensetzung.

Typische Beispiele für **nichtionische grenzflächenaktive Stoffe** sind Fettalkoholpolyglycolether, Polyglycerinester, Alkylphenolpolyglycolether, Fettsäurepolyglycolester, Fettsäureamid-polyglycolether, Fettaminpolyglycolether, alkoxylierte Mono-/Di-/Triglyceride, Mischether bzw. Mischformale, Alk(en)yloligoglykoside bzw. Glucoronsäurederivate - gegebenenfalls partiell oxidiert, Fettsäure-N-alkylglucamide, Proteinhydrolysate (insbesondere pflanzliche Produkte auf Weizenbasis), Polyolfettsäureester, Zuckerester, Sorbitanester, Polysorbate und Aminoxide. Sofern die nichtionischen Tenside Polyglycoletherketten enthalten, können diese eine konventionelle, vorzugsweise jedoch eine eingeengte Homologenverteilung aufweisen. In einer besonders bevorzugten Ausführungsform ist der nichtionische grenzflächenaktive Stoff ausgewählt aus der Gruppe der Alk(en)yloligoglycoside.

Alk(en)yloligoglykoside stellen bekannte nichtionische Tenside dar, die der Formel (**I**) folgen,

**R¹O-[G]**ₚ (I)

in der R¹ für einen Alk(en)ylrest, G für einen Zuckerrest mit 5 oder 6 Kohlenstoffatomen und p für Zahlen von 1 bis 10 steht. Sie können nach den einschlägigen Verfahren der präparativen organischen Chemie erhalten werden. Stellvertretend für das umfangreiche Schrifttum sei hier auf die EP0301298A1 und WO 90/03977 Averwiesen.

Die Alk(en)yloligoglykoside können sich von Aldosen bzw. Ketosen mit 5 oder 6 Kohlenstoffatomen, vorzugsweise der Glucose, ableiten. Die bevorzugten Alk(en)yloligoglykoside sind somit Alk(en)yloligoglucoside. Die Indexzahl p in der allgemeinen Formel (I) gibt den Oligomerisierungsgrad (DP-Grad) an, d. h. die Verteilung von Mono- und Oligoglykosiden, und steht für eine Zahl zwischen 1 und 10. Während p in einer gegebenen Verbindung stets ganzzahlig sein muss und hier vor allem die Werte p = 1 bis 6 annehmen kann, ist der Wert p für ein bestimmtes Alk(en)yloligoglykosid eine analytisch ermittelte rechnerische Größe, die meistens eine gebrochene Zahl darstellt. Vorzugsweise werden Alk(en)yloligoglykoside mit einem mittleren Oligomerisierungsgrad p von 1,1 bis 3,0 eingesetzt. Aus anwendungstechnischer Sicht sind solche Alk(en)yloligoglykoside bevorzugt, deren Oligomerisierungsgrad kleiner als 1,7 ist und insbesondere zwischen 1,2 und 1,4 liegt. Erfindungsgemäß bevorzugt werden Alkyloligoglykoside verwendet, in denen sich der Rest R¹ von primären Alkoholen mit 8 bis 24 Kohlenstoffatomen, insbesondere 12 bis 24 und besonders bevorzugt 16 bis 18 Kohlenstoffatomen ableitet. Es können auch technische Gemische der Alkohole eingesetzt werden.

Als oberflächenaktive Stoffe können auch anionische, kationische und/oder amphotere bzw. zwitterionische Tenside/Emulgatoren oder ein Gemisch dieser Tenside/Emulgatoren in den erfindungsgemäßen Zusammensetzungen enthalten sein.

Typische Beispiele für **anionische Tenside** sind Seifen, Alkylbenzolsulfonate, Alkansulfonate, Olefinsulfonate, Alkylethersulfonate, Glycerinethersulfonate, α-Methylestersulfonate, Sulfofettsäuren, Alkylsulfate, Fettalkoholethersulfate, Glycerinethersulfate, Fettsäureethersulfate, Hydroxymischethersulfate, Monoglycerid(ether)sulfate, Fettsäureamid(ether)sulfate, Mono- und Dialkylsulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, N-Acylaminosäuren, wie beispielsweise Acyllactylate, Acyltartrate, Acylglutamate und Acylaspartate, Alkyloligoglucosidsulfate, Proteinfettsäurekondensate (insbesondere pflanzliche Produkte auf Weizenbasis) und Alkyl(ether)phosphate. Sofern die anionischen Tenside Polyglycoletherketten enthalten, können diese eine konventionelle, vorzugsweise jedoch eine eingeengte Homologenverteilung aufweisen. Typische Beispiele für **kationische Tenside** sind quartäre Ammoniumverbindungen, wie beispielsweise das Dimethyldistearylammoniumchlorid, und Esterquats, insbesondere quaternierte Fettsäuretrialkanolaminestersalze. Typische Beispiele für **amphotere bzw. zwitterionische Tenside** sind Alkylbetaine, Alkylamidobetaine, Aminopropionate, Aminoglycinate, Imidazoliniumbetaine und Sulfobetaine. Bei den genannten Tensiden handelt es sich ausschließlich um bekannte Verbindungen. Hinsichtlich Struktur und Herstellung dieser Stoffe sei auf einschlägige Übersichtsarbeiten auf diesem Gebiet verwiesen. Typische Beispiele für besonders geeignete milde, d.h. besonders hautverträgliche Tenside sind Fettalkoholpolyglycolethersulfate, Monoglyceridsulfate, Mono- und/oder Dialkylsulfosuccinate, Fettsäureisethionate, Fettsäuresarcosinate, Fettsäuretauride, Fettsäureglutamate, α-Olefinsulfonate, Ethercarbonsäuren, Alkyloligoglucoside, Fettsäureglucamide, Alkylamidobetaine, Amphoacetale und/oder Proteinfettsäurekondensate, letztere vorzugsweise auf Basis von Weizenproteinen.

Eine weitere bevorzugte Zusammensetzung ist dadurch gekennzeichnet, dass sie einen Gehalt an folgenden Komponenten aufweist: (a) 0,1 -10 Gew.-% wenigstens einer Wachskomponente gemäß einem der Ansprüche 1 bis 4, (b) 1 - 25 Gew.-% wenigstens einer bei 25 °C flüssigen Ölkomponente, (c) 0,1 - 5 Gew.-% eines C12-C24-Fettalkohols oder C12-C24-Partialglycerids oder eines beliebigen Gemisches davon, (d) 0,5-10 Gew.-% eines C8 - C24-Alkyloligoglucosids und (e) Wasser. Besonders bevorzugt ist eine Zusammensetzung mit folgendem Gehalt: (a) 0,5 -5 Gew.-% wenigstens einer Wachskomponente gemäß einem der Ansprüche 1 bis 4, (b) 5 - 15 Gew.-% wenigstens einer bei 20 °C flüssigen Ölkomponente, (c) 1 - 5 Gew.-% eines C12-C24-Fettalkohols oder C12-C24-Partialglycerids oder eines beliebigen Gemisches davon, (d) 0,5 - 5 Gew.-% eines C12 - C24-Alkyloligoglucosids und (e) bis zu 90 Gew.-% Wasser. Ester des Pentaerythrits, die durch Umsetzung von Pentaerythrit mit einem Fettsäuregemisch enthaltend 42 - 48 Gew.-% C16-Fettsäure und 50 - 56 Gew.-% C18-Fettsäure (restliche Mengen: ≤ C14-Fettsäuren und > C18-Fettsäuren) erhalten werden und 12 - 19 Gew.-% Monoester, (b) 25 - 35 Gew.-% Diester, (c) 30 - 40 Gew.-% Triester und (d) 6 - 11 Gew.-% Tetraester aufweisen, sind als Wachskomponente (a) erfindungsgemäß bevorzugt.

### Hydrotrope

Eine weitere bevorzugte Ausführungsform der Erfindung enthält zusätzlich wenigstens eine Verbindung aus der Gruppe der Hydrotrope. Diese dienen der Verbesserung des Fließverhaltens und Sensorik. Hierfür können beispielsweise Ethanol, Isopropylalkohol, oder Polyole eingesetzt werden. Polyole, die hier in Betracht kommen, besitzen vorzugsweise 2 bis 15, vorzugsweise 2 bis 6 Kohlenstoffatome und mindestens zwei Hydroxylgruppen. Erfindungsgemäß bevorzugt ist Glycerin. Der Gehalt des Hydrotrops liegt zwischen 0,1 - 10 Gew.-%, vorzugsweise 0,5 - 5 Gew.-% und insbesondere 1 - 5 Gew.-% bezogen auf die Gesamtzusammensetzung.

### Weitere fakultative Hilfs- und Zusatzstoffe

Je nach Applikationszweck enthalten die kosmetischen Formulierungen eine Reihe weiterer Hilfs- und Zusatzstoffe wie beispielsweise Verdickungsmittel, Überfettungsmittel, Stabilisatoren, Polymere, Lecithine, Phospholipide, biogene Wirkstoffe, UV-Lichtschutzfaktoren, Antioxidantien, Deodorantien, Filmbildner, Quellmittel, Insektenrepellentien, Hydrotrope, Solubilisatoren, Konservierungsmittel, Parfümöle, Farbstoffe etc., die nachstehend exemplarisch aufgelistet sind. Die Mengen der jeweiligen Zusätze richten sich nach der beabsichtigten Verwendung.

Als **Verdickungsmittel** eignen sich beispielsweise Aerosil-Typen (hydrophile Kieselsäuren), Polysaccharide, insbesondere Xanthan-Gum, Guar-Guar, Agar-Agar, Alginate und Tylosen, Carboxymethylcellulose und Hydroxyethyl- und Hydroxypropylcellulose, Polyvinylalkohol, Polyvinylpyrrolidon und Bentonite wie z. B. Bentone^{®} Gel VS-5PC (Rheox).

Unter **UV-Lichtschutzfaktoren** sind beispielsweise bei Raumtemperatur flüssig oder kristallin vorliegende organische Substanzen (Lichtschutzfilter) zu verstehen, die in der Lage sind, ultraviolette Strahlen zu absorbieren und die aufgenommene Energie in Form längerwelliger Strahlung, z. B. Wärme wieder abzugeben. UV-B-Filter können öllöslich oder wasserlöslich sein. Als typische UV-A-Filter kommen insbesondere Derivate des Benzoylmethans in Frage. Die UV-A und UV-B-Filter können selbstverständlich auch in Mischungen eingesetzt werden, z.B. Kombinationen aus den Derivaten des Benzoylmethans, z. B. 4-tert.-Butyl-4'-methoxydibenzoylmethan (Parsol^{®} 1789) und 2-Cyano-3,3-phenylzimtsäure-2-ethyl-hexylester (Octocrylene) sowie Estern der Zimtsäure, vorzugsweise 4-Methoxyzimtsäure-2-ethylhexylester und/oder 4-Methoxyzimtsäurepropylester und/oder 4-Methoxyzimtsäureisoamylester. Häufig werden derartige Kombinationen mit wasserlöslichen Filtern wie z.B. 2-Phenylbenzimidazol-5-sulfonsäure und deren Alkali-, Erdalkali-, Ammonium-, Alkylammonium-, Alkanolammonium- und Glucammoniumsalze kombiniert.

Neben den genannten löslichen Stoffen kommen auch unlösliche Lichtschutzpigmente, nämlich feindisperse Metalloxide in Frage. Beispiele für geeignete Metalloxide sind insbesondere Zinkoxid und Titandioxid.

Neben den beiden vorgenannten Gruppen primärer Lichtschutzstoffe können auch sekundäre Lichtschutzmittel vom Typ der Antioxidantien eingesetzt werden, die die photochemische Reaktionskette unterbrechen, welche ausgelöst wird, wenn UV-Strahlung in die Haut eindringt.

Unter **biogenen Wirkstoffen** sind beispielsweise Tocopherol, Tocopherolacetat, Tocopherolpalmitat, Ascorbinsäure, (Desoxy)Ribonucleinsäure und deren Fragmentierungsprodukte, β-Glucane, Retinol, Bisabolol, Allantoin, Phytantriol, Panthenol, AHA-Säuren, Aminosäuren, Ceramide, Pseudoceramide, essentielle Öle, Pflanzenextrakte, wie z. B. Prunusextrakt, Bambaranussextrakt und Vitaminkomplexe zu verstehen.

**Desodorierende Wirkstoffe** wirken Körpergerüchen entgegen, überdecken oder beseitigen sie. Körpergerüche entstehen durch die Einwirkung von Hautbakterien auf apokrinen Schweiß, wobei unangenehm riechende Abbauprodukte gebildet werden. Dementsprechend eignen sich als deosodorierende Wirkstoffe u.a. keimhemmende Mittel, Enzyminhibitoren, Geruchsabsorber oder Geruchsüberdecker.

Als **Insekten-Repellentien** kommen beispielsweise N,N-Diethyl-m-toluamid, 1,2-Pentandiol oder 3-(N-n-Butyl-N-acetyl-amino)-propionsäureethylester), welches unter der Bezeichnung Insect Repellent^{®} 3535 von der Merck KGaA vertrieben wird, sowie Butylacetylaminopropionate in Frage.

Als Selbstbräuner eignet sich Dihydroxyaceton. Als Tyrosinhinbitoren, die die Bildung von Melanin verhindern und Anwendung in Depigmentierungsmitteln finden, kommen beispielsweise Arbutin, Ferulasäure, Kojisäure, Cumarinsäure und Ascorbinsäure (Vitamin C) in Frage.

Als **Konservierungsmittel** eignen sich beispielsweise Phenoxyethanol, Formaldehydlösung, Parabene, Pentandiol oder Sorbinsäure sowie die unter der Bezeichnung Surfacine® bekannten Silberkomplexe und die in Anlage 6, Teil A und B der Kosmetikverordnung aufgeführten weiteren Stoffklassen.

Als **Parfümöle** seien genannt Gemische aus natürlichen und synthetischen Riechstoffen. Natürliche Riechstoffe sind Extrakte von Blüten, Stengeln und Blättern, Früchten, Fruchtschalen, Wurzeln, Hölzern, Kräutern und Gräsern, Nadeln und Zweigen, Harzen und Balsamen. Weiterhin kommen tierische Rohstoffe, wie beispielsweise Zibet und Castoreum sowie synthetische Riechstoffverbindungen vom Typ der Ester, Ether, Aldehyde, Ketone, Alkohole und Kohlenwasserstoffe in Frage.

Als **Farbstoffe** können die für kosmetische Zwecke geeigneten und zugelassenen Substanzen verwendet werden. Beispiele sind Kochenillerot A (C.I. 16255), Patentblau V (C.I.42051), Indigotin (C.I.73015), Chlorophyllin (C.I.75810), Chinolingelb (C.I.47005), Titandioxid (C.I.77891), Indanthrenblau RS (C.I. 69800) und Krapplack (C.I.58000). Diese Farbstoffe werden üblicherweise in Konzentrationen von 0,001 bis 0,1 Gew.-%, bezogen auf die gesamte Mischung, eingesetzt.

### Beispiele

Die angegebenen Mengen beziehen sich auf Gew.-% der handelsüblichen Substanz in der Gesamtzusammensetzung. V1 ist ein Vergleichsbeispiel.

| **Handelsname** (INCI) | **V1** | **1** |
|---|---|---|
| **Emulgade® PL 68/50** (Cetearyl Glucosid (und) Cetearylalkohol) | 2,7 | 2,7 |
| Pentaerythrit-C18-ester¹⁾ | 1,6 | |
| Pentaerythrit-C16/18-ester²⁾ | | 1,6 |
| **Cetiol® J600** | 3,0 | 3,0 |
| (Oleyl Erucat) | | |
| **Myritol® 312** | 4,0 | 4,0 |
| (Caprylic/Capric Triglycerid) | | |
| **Cetiol® V** | 2,0 | 2,0 |
| (Decyl Oleat) | | |
| **Cetiol® OE** | 2,0 | 2,0 |
| (Dicaprylyl Ether) | | |
| **Wacker® AK 350** (Dimethicone) | 0,5 | 0,5 |
| Glycerin | 3,0 | 3,0 |
| Wasser, Konservierungsmittel q.s. | ad 100 | |
| Kaliumhydroxid | pH 7,0 | |
| | | |

| **Viskositätsdaten³⁾ bei 20 °C [Pa·s]** | | |
|---|---|---|
| nach Vorbereitung | 12,4 | 12,4 |
| nach 1 Woche | 11,2 | 9,2 |
| nach 2 Wochen | 10,0 | 7,6 |
| nach 4 Wochen | 8,0 | 8,4 |
| nach 8 Wochen | 6,4 | 8,0 |
| nach 12 Wochen | 2,0 | 8,0 |

| **Phasenstabilität⁴⁾ bei -5°C/20 °C /40°C** | | |
|---|---|---|
| nach 1 Woche | 1/1/1 | 1/1/1 |
| nach 2 Wochen | 1/1/1 | 1/1/1 |
| nach 4 Wochen | 1/2/2 | 1/1/1 |
| nach 8 Wochen | 5/2/3 | 1/1/1 |
| nach 12 Wochen | 5/5/5 | 1/1/1 |

| **Makroskopische⁵⁾/ Mikroskopische⁶⁾Erscheinung bei 20 °C** | | |
|---|---|---|
| nach Vorbereitung | 1/2 | 1/2 |
| nach 1 Woche | 1/2 | 1/2 |
| nach 2 Wochen | 2/3 | 1/2 |
| nach 4 Wochen | 3/3 | 1/2 |
| nach 8 Wochen | 4/3 | 1/2 |
| nach 12 Wochen | 4/4 | 1/2 |

| **Sensorische Bewertung⁷⁾** | | |
|---|---|---|
| nach Vorbereitung | 1 | 1 |
| nach 12 Wochen | 3(stumpf) | 1 |

| | | |
|---|---|---|
| 1) Ester des Pentaerythrits, der >90 Gew.-% C18-Fettsäure aufweist. 2) Ester des Pentaerythrits, der durch Umsetzung von 1 Mol Pentaerythrit mit ca. 2 Mol eines Fettsäuregemisches aus 42 - 48 Gew.-% C16-Fettsäure und 50 - 56 Gew.-% C18-Fettsäure (restliche Mengen: ≤ C14-Fettsäuren und > C18-Fettsäuren) erhalten wird und folgende Esterverteilung aufweist: 12 - 19 Gew.-% Monoester, (b) 25 - 35 Gew.-% Diester, (c) 30 - 40 Gew.-% Triester und (d) 6 - 11 Gew.-% Tetraester und weniger als 0,3 Gew.-% C17-Fettsäureacylgruppen enthält. 3) Viskositätsmessungen: Brookfield RVF, Spindel 5, 10 Umdrehungen pro Minute, 23°C. 4) Bewertungskriterien für die visuelle Phasenstabilität: 1 = stabil; 2 = geringe Separation; 3 = Separation; 4 = deutliche Separation; 5 = Trennung. 5) Bewertungskriterien zum visuellen makroskopischen Erscheinungsbild: 1 = glatt und glänzend; 2 = glatt und matt; 3 = matt; 3 = grobe Struktur; 4 = sichtbare Rekristallisate Die Bewertung der Formulierungen erfolgte nach Temperieren auf RT. 6) Bewertungskriterien zum mikroskopischen Erscheinungsbild: 1 = durchschnittliche Teilchengröße ≤ 1µm; 2 = durchschnittliche Teilchengröße 1 - 4µm; 3 = durchschnittliche Teilchengröße 4 - 13µm; 4 = durchschnittliche Teilchengröße 13 - 20µm; 5 = durchschnittliche Teilchengröße 20 - 50µm. Die Partikelgröße der Testemulsion wurde visuell mit der Partikelgröße von Standardemulsionen verglichen. Zur Bestimmung der Partikelgröße der Standardemulsionen wird mittels Laserbeugung ein Beugungsmuster ermittelt. Aus den Lichtintensitäten dieser Beugungsmuster wird dann mittels der Fraunhofer-Theorie die Teilchengrößenverteilung errechnet (Sympatec Helos). 7) Sensorische Bewertungskriterien Testgruppe: 10 erfahrene und geschulte Freiwillige; 1 = sehr hohe Akzeptanz; 2 = durchschnittliche Akzeptanz; 3 = nicht akzeptabel 10µl der o.g. Zusammensetzungen, die vorher auf 20 °C gebracht wurden, wurden mittels einer Mikropipette auf die unbehaarte Seite der Unterarme der Probanden appliziert und mit den Fingern der Hände der kontralateralen Seite eingerieben. Die Beurteilung der Sensorik erfolgte während und nach der Absorption. Der Sensorik-Test wurde an 10 Probanden durchgeführt, wie in dem Buch "Cosmetic Lipids and the Skin Barrier" (Marcel Dekker Verlag New York, 2002, Ed. Thomas Förster, S. 319-352) beschrieben. | | |

**Tabelle 2**

| Die angegebenen Mengen beziehen sich auf Gew.-% der handelsüblichen Substanz in der Gesamtzusammensetzung. V2 bis V4 sind Vergleichsbeispiele. | | | | | |
|---|---|---|---|---|---|
| **Handelsname** (INCI) | **V2** | **V3** | **V4** | **2** | **3** |
| **Emulgade® PL 68/50** | 2,7 | 2,7 | 2,7 | 2,7 | 2,7 |
| (Cetearyl Glucosid, Cetearylalkohol) | | | | | |
| Pentaerythrityl Distearat¹⁾ | | | | 1,6 | 1,6 |
| **Lanette® O** | 1,6 | 1,6 | | | |
| (Cetearvlalkohol) | | | | | |
| **Cutina® GMS-V** | | | 1.6 | | |
| (Glyceryl Monostearat) | | | | | |
| **Cetiol® J600** | 3,0 | 3,0 | 3,0 | 3,0 | 3,0 |
| (Oleylerucat) | | | | | |
| **Myritol® 312** | 4,0 | 4,0 | 4,0 | 4,0 | 4,0 |
| (Caprylic/Capric Triglycerid) | | | | | |
| **Cetiol® V** | 2.0 | 2.0 | 2.0 | 2.0 | 2.0 |
| (Decvl Oleat) | | | | | |
| **Cetiol® OE** | 2.0 | 2.0 | 2.0 | 2.0 | 2.0 |
| (Dicaprylyl Ether) | | | | | |
| **Wacker-Silikonöl AK® 350** | 0,5 | | 0,5 | 0,5 | |
| (Dimethicon) | | | | | |
| Glycerin | 3,0 | 3,0 | 3,0 | 3,0 | 3,0 |
| Wasser | ad 100 | ad 100 | ad 100 | ad 100 | ad 100 |
| | | | | | |
| **Sensorische Beurteilung²⁾** | | | | | |
| Verteilbarkeit | 0 | 0 | 0 | + | ++ |
| Weiße Rückstandbildung | + | 0 | ++ | ++ | + |
| Absorption | - | - | + | ++ | ++ |
| Klebrigkeit | 0 | 0 | + | + | + |
| Glätte | 0 | - | 0 | ++ | ++ |
| Weichheit | - | - | + | ++ | ++ |
| Akzeptanz | - | - | + | + | ++ |

| | | | | | |
|---|---|---|---|---|---|
| 1) Ester des Pentaerythrits, der durch Umsetzung von 1 Mol Pentaerythrit mit ca. 2 Mol eines Fettsäuregemisches aus 42 - 48 Gew.-% C16-Fettsäure und 50 - 56 Gew.-% C18-Fettsäure (restliche Mengen: ≤ C14-Fettsäuren und > C18-Fettsäuren) erhalten wird und folgende Esterverteilung aufweist: 12 - 19 Gew.-% Monoester, (b) 25 - 35 Gew.-% Diester, (c) 30 - 40 Gew.-% Triester und (d) 6 - 11 Gew.-% Tetraester und weniger als 0,3 Gew.-% C17-Fettsäureacylgruppen enthält. | | | | | |

### Sensorische Bewertungskriterien im Vergleich zu Formulierung V2 - V4.

Test Gruppe: 10 erfahrene und ausgebildete Freiwillige; ++ = hervorragend; + = sehr gut; 0 = gut; - = mittelmäßig; - - = schlecht; Vorgehensweise wie unter Punkt 7) bei Tabelle 1 beschrieben.

## Patentansprüche

1. Kosmetische und/oder pharmazeutische Zusammensetzung, enthaltend ein Gemisch von Estern von C6-C22-Fettsäuren des Pentaerythrits, die weniger als 0.3 Gew.-% C17-Fettsäureacylgruppen enthalten und einen Schmelzpunkt von wenigstens 30°C aufweisen, mit einem Anteil an (a) 5 - 35 Gew.% Monoester, (b) 20 - 50 Gew.-% Diester und (c) 25 - 50 Gew.-% Triester, und ggf. Tetraester, in einer Menge von 0,1- 20 Gew.-%.

2. Zusammensetzung gemäß Anspruch 1, **dadurch gekennzeichnet, dass** sie frei von Siliconölen ist.

3. Zusammensetzung gemäß wenigstens einem der Ansprüche 1 bis 2, **dadurch gekennzeichnet, dass** zusätzlich wenigstens eine weitere Wachskomponente enthalten ist.

4. Zusammensetzung gemäß Anspruch 3, **dadurch gekennzeichnet, dass** die weitere Wachskomponente ausgewählt ist aus der Gruppe der C12-C24-Fettalkohole und/oder der C12-C24-Partialglyceride.

5. Zusammensetzung gemäß wenigstens einem der Ansprüche 1 bis 4, **dadurch gekennzeichnet, dass** zusätzlich wenigstens ein nichtionischer grenzflächenaktiver Stoff enthalten ist.

6. Zusammensetzung gemäß Anspruch 5, **dadurch gekennzeichnet, dass** der nichtionische grenzflächenaktive Stoff ausgewählt ist aus der Gruppe der Alk(en)yloligoglycoside.

7. Zusammensetzung gemäß wenigstens einem der Ansprüche 1 bis 6, **dadurch gekennzeichnet, dass** zusätzlich wenigstens eine bei 25°C flüssige Ölkomponente enthalten ist.

8. Zusammensetzung gemäß Anspruch 1 , **dadurch gekennzeichnet, dass** sie wenigstens
(a) 0,1 -10 Gew.-% wenigstens einer Wachskomponente von Estern von C6-C22-Fettsäuren des Pentaerythrits, die weniger als 0.3 Gew.-% C17-Fettsäureacylgruppen enthalten und einen Schmelzpunkt von wenigstens 30°C aufweisen, mit einem Anteil an (a) 5 - 35 Gew.% Monoester, (b) 20 - 50 Gew.-% Diester und (c) 25 - 50 Gew.-% Triester und ggf. Tetraester,
(b) 1 - 25 Gew.-% wenigstens einer bei 25°C flüssigen Ölkomponente,
(c) 0,1-5 Gew.-% eines C12 - C24-Fettalkohols oder C12-C24-Partialglycerids oder eines beliebigen Gemisches davon
(d) 0,5 -10 Gew.-% eines C12 -C24-Alkyloligoglucosids und
(e) Wasser
enthält.

9. Zusammensetzung gemäß wenigstens einem der Ansprüche 1 bis 8, **dadurch gekennzeichnet, dass** man für die Veresterung ein Fettsäuregemisch enthaltend 40 - 50 Gew.-% C16-Fettsäure und 45 - 55 Gew.-% C18-Fettsäure einsetzt.

10. Ester von C6-C22-Fettsäuren des Pentaerythrits, die weniger als 0.3 Gew.-% C17-Fettsäureacylgruppen enthalten und einen Schmelzpunkt von wenigstens 30° C aufweisen und a) 12 - 19 Gew.-% Monoester, (b) 25 - 35 Gew.-% Diester, (c) 30 - 40 Gew.-% Triester und (d) 6 - 11 Gew.-% Tetraester, enthalten.

11. Ester gemäß Anspruch 10, **dadurch gekennzeichnet, dass** man für die Veresterung ein Fettsäuregemisch enthaltend 40 - 50 Gew.-% C16-Fettsäure und 45 - 55 Gew.-% C18-Fettsäure einsetzt.

12. Verfahren zur Herstellung von C16/C18-Fettsäure-Pentaerythritestern gemäß wenigstens einem der Ansprüche 10 bis 11, wobei man pro 1 Mol Pentaerythrit 1.8 - 2.2 Mol eines Fettsäuregemisches enthaltend 40 - 50 Gew.-% C16-Fettsäure und 45 - 55 Gew.-% C18-Fettsäure oder ein Rohstoffgemisch mit entsprechender Fettsäureverteilung einsetzt und (a) die Veresterung bei Temperaturen im Bereich von 180°C bis 250°C unter Schutzgasatmosphäre, und ohne Lösungsmittel durchführt, (b) das entstehende Wasser abdestilliert, (c) das erhaltene Reaktionsgemisch im Vakuum solange nachrührt, bis eine Säurezahl von <1 und eine OH-Zahl von 145 - 158 erreicht ist, (d) nicht abreagiertes Pentaerythrit abfiltriert und (e) ggf. eine Nachbehandlung mit Wasserstoffperoxid durchführt.

13. Verwendung von Estern von C6-C22-Fettsäuren des Pentaerythrits, die weniger als 0.3 Gew.-% C17-Fettsäureacylgruppen enthalten und einen Schmelzpunkt von wenigstens 30° C aufweisen, mit einem Anteil an (a) 5 - 35 Gew.-% Monoester, (b) 20 - 50 Gew.-% Diester und (c) 25 - 50 Gew.-% Triester, und ggf. Tetraester in kosmetischen und/oder pharmazeutischen Zusammensetzungen.

14. Verwendung gemäß Anspruch 13, **dadurch gekennzeichnet, dass** man für die Veresterung ein Fettsäuregemisch enthaltend 40 - 50 Gew.-% C16-Fettsäure und 45 - 55 Gew.-% C18- Fettsäure einsetzt.

15. Verwendung gemäß wenigstens einem der Ansprüche 13 bis 14, **dadurch gekennzeichnet, dass** die Ester (a) 10 - 25 Gew.-% Monoester, (b) 25 - 40 Gew.-% Diester und (c) 30 - 45 Gew.-% Triester und ggf. Tetraester enthalten.

16. Verwendung nach Anspruch 15, **dadurch gekennzeichnet, dass** die Ester (a) 12-19 Gew.-% Monoester, (b) 25 -35 Gew.-% Diester, (c) 30 - 40 Gew.-% Triester und (d) 6 -11 Gew.-% Tetraester enthalten.

17. Verwendung eines Esters gemäß einem der Ansprüche 10 bis 11 als Konsistenzgeber.

## Claims

1. A cosmetic and/or pharmaceutical composition comprising a mixture of C6-C22 fatty acid esters of pentaerythritol, which comprise less than 0.3% by weight of C17 fatty acid acyl groups and have a melting point of at least 30°C and which have a proportion of (a) 5 - 35% by weight of monoester, (b) 20 - 50% by weight of diester and (c) 25 - 50% by weight of triester and optionally tetraester in an amount of 0.1 - 20% by weight.

2. The composition according to claim 1, wherein said composition is free from silicone oils.

3. The composition according to at least one of claims 1 and 2, wherein at least one further wax component is also present.

4. The composition according to claim 3, wherein the further wax component is selected from the group of C12 - C24 fatty alcohols and/or C12 - C24 partial glycerides.

5. The composition according to at least one of claims 1 to 4, wherein at least one nonionic surface-active substance is also present.

6. The composition according to claim 5, wherein the nonionic surface-active substance is selected from the group of alk(en)yl oligoglycosides.

7. The composition according to at least one of claims 1 to 6, wherein at least one oil component liquid at 25°C is also present.

8. The composition according to claim 1, wherein said composition comprises at least
(a) 0.1 - 10% by weight of at least one wax component of C6 - C22 fatty acid esters of pentaerythritol, which comprise less than 0.3% by weight of C17 fatty acid acyl groups and have a melting point of at least 30°C and which have a proportion of (a) 5 - 35% by weight of monoester, (b) 20 - 50% by weight of diester and (c) 25 - 50% by weight of triester and optionally tetraester,
(b) 1 - 25% by weight of at least one oil component liquid at 25°C,
(c) 0.1 - 5% by weight of a C12 - C24 fatty alcohol or C12 - C24 partial glyceride or any mixture thereof,
(d) 0.5 - 10% by weight of a C12 - C24 alkyl oligoglucoside and
(e) water.

9. The composition according to at least one of claims 1 to 8, wherein a fatty acid mixture comprising 40 - 50% by weight of C16 fatty acid and 45 - 55% by weight of C18 fatty acid is used for the esterification.

10. A C6-C22 fatty acid ester of pentaerythritol, which comprises less than 0.3% by weight of C17 fatty acid acyl groups and has a melting point of at least 30°C and which comprises (a) 12 - 19% by weight of monoester, (b) 25 - 35% by weight of diester, (c) 30 - 40% by weight of triester and (d) 6 - 11% by weight of tetraester.

11. The ester according to claim 10, wherein a fatty acid mixture comprising 40 - 50% by weight of C16 fatty acid and 45 - 55% by weight of C18 fatty acid is used for the esterification.

12. A method for preparing C16/C18 fatty acid pentaerythritol esters according to at least one of claims 10 and 11, wherein 1.8 - 2.2 mol of a fatty acid mixture comprising 40 - 50% by weight of C16 fatty acid and 45 - 55% by weight of C18 fatty acid or a crude mixture having appropriate fatty acid distribution is used per 1 mol of pentaerythritol and (a) the esterification is carried out at temperatures in the range of 180°C to 250°C under a protective gas atmosphere and without solvent, (b) the water formed is distilled off, (c) the resulting reaction mixture is subsequently stirred under vacuum until an acid number of <1 and an OH number of 145 - 158 is reached, (d) unreacted pentaerythritol is filtered off and (e) optionally a subsequent treatment with hydrogen peroxide is carried out.

13. The use of C6 - C22 fatty acid esters of pentaerythritol, which comprise less than 0.3% by weight of C17 fatty acid acyl groups and have a melting point of at least 30°C and which have a proportion of (a) 5 - 35% by weight of monoester, (b) 20 - 50% by weight of diester and (c) 25 - 50% by weight of triester and optionally tetraester in cosmetic and/or pharmaceutical compositions.

14. The use according to claim 13, wherein a fatty acid mixture comprising 40 - 50% by weight of C16 fatty acid and 45 - 55% by weight of C18 fatty acid is used for the esterification.

15. The use according to at least one of claims 13 and 14, wherein the esters comprise (a) 10 - 25% by weight of monoester, (b) 25 - 40% by weight of diester and (c) 30 - 45% by weight of triester and optionally tetraester.

16. The use according to claim 15, wherein the esters comprise (a) 12 - 19% by weight of monoester, (b) 25 - 35% by weight of diester, (c) 30 - 40% by weight of triester and (d) 6 - 11% by weight of tetraester.

17. The use of an ester according to either of claims 10 and 11 as bodying agent.

## Revendications

1. Composition cosmétique et/ou pharmaceutique, contenant un mélange d'esters d'acides gras en C₆-C₂₂ du pentaérythritol, qui contiennent moins de 0,3% en poids de groupes acyle d' acides gras en C₁₇ et qui présentent un point de fusion d'au moins 30°C, présentant une proportion (a) de 5-35% en poids de monoesters, (b) de 20-50% en poids de diesters et (c) de 25-50% en poids de triesters, et le cas échéant des tétraesters, en une quantité de 0,1-20% en poids.

2. Composition selon la revendication 1, **caractérisée en ce qu'**elle est exempte d'huiles siliconées.

3. Composition selon l'une quelconque des revendications 1 à 2, **caractérisée en ce qu'**elle contient en outre au moins un autre composant de type cire.

4. Composition selon la revendication 3, **caractérisée en ce que** l'autre composant de type cire est choisi dans le groupe des alcools gras en C₁₂-C₂₄ et/ou des glycérides partiels en C₁₂-C₂₄.

5. Composition selon au moins l'une quelconque des revendications 1 à 4, **caractérisée en ce qu'**elle contient en outre au moins une substance tensioactive non ionique.

6. Composition selon la revendication 5, **caractérisée en ce que** la substance tensioactive non ionique est choisie dans le groupe des alkyl/alcényloligoglycosides.

7. Composition selon au moins l'une quelconque des revendications 1 à 6, **caractérisée en ce qu'**elle contient en outre au moins un composant huileux liquide à 25°C.

8. Composition selon la revendication 1, **caractérisée en ce qu'**elle contient au moins
(a) 0,1-10% en poids d'au moins un composant de type cire d'esters d'acides gras en C₆-C₂₂ du pentaérythritol, qui contiennent moins de 0,3% en poids de groupes acyle d' acides gras en C₁₇ et qui présentent un point de fusion d'au moins 30°C, présentant une proportion (a) de 5-35% en poids de monoesters, (b) de 20-50% en poids de diesters et (c) de 25-50% en poids de triesters, et le cas échéant des tétraesters,
(b) 1-25% en poids d'au moins un composant huileux liquide à 25°C,
(c) 0,1-5% en poids d' un alcool gras en C₁₂-C₂₄ ou d'un glycéride partiel en C₁₂-C₂₄ ou d'un mélange quelconque de ceux-ci,
(d) 0,5-10% en poids d'un C₁₂-C₂₄-alkyloligoglucoside, et
(e) de l'eau.

9. Composition selon au moins l'une quelconque des revendications 1 à 8, **caractérisée en ce qu'**on utilise, pour l'estérification, un mélange d'acides gras contenant 40-50% en poids d'acides gras en C₁₆ et 45-55% en poids d'acides gras en C₁₈.

10. Esters d'acides gras en C₆-C₂₂ du pentaérythritol, qui contiennent moins de 0,3% en poids de groupes acyle d' acides gras en C₁₇ et qui présentent un point de fusion d'au moins 30°C, et (a) 12-19% en poids de monoesters, (b) 25-35% en poids de diesters, (c) 30-40% en poids de triesters et (d) 6-11% en poids de tétraesters.

11. Esters selon la revendication 10, **caractérisés en ce qu'**on utilise, pour l'estérification, un mélange d'acides gras contenant 40-50% en poids d'acides gras en C₁₆ et 45-55% en poids d'acides gras en C₁₈.

12. Procédé pour la préparation d'esters d'acides gras en C₁₆/C₁₈ du pentaérythritol selon au moins l' une quelconque des revendications 10 à 11, dans lequel on utilise, par 1 mole de pentaérythritol, 1,8-2,2 moles d'un mélange d'acides gras contenant 40-50% en poids d'acides gras en C₁₆ et 45-55% en poids d'acides gras en C₁₈ ou un mélange de matières premières présentant une répartition correspondante des acides gras et (a) on réalise l'estérification à des températures dans la plage de 180°C à 250°C sous une atmosphère de gaz de protection et sans solvant, (b) on élimine l'eau formée par distillation, (c) on agite le mélange réactionnel obtenu sous vide jusqu'à ce qu'on atteigne un indice d'acide < 1 et un indice d'OH de 145-158, (d) on élimine le pentaérythritol qui n'a pas réagi par filtration et (e) on réalise le cas échéant un post-traitement avec du peroxyde d'hydrogène.

13. Utilisation d'esters d'acides gras en C₆-C₂₂ du pentaérythritol, qui contiennent moins de 0,3% en poids de groupes acyle d' acides gras en C₁₇ et qui présentent un point de fusion d'au moins 30°C, présentant une proportion (a) de 5-35% en poids de monoesters, (b) de 20-50% en poids de diesters et (c) de 25-50% en poids de triesters, et le cas échéant des tétraesters dans des compositions cosmétiques et/ou pharmaceutiques.

14. Utilisation selon la revendication 13, **caractérisée en ce qu'**on utilise, pour l'estérification, un mélange d'acides gras contenant 40-50% en poids d'acides gras en C₁₆ et 45-55% en poids d' acides gras en C₁₈.

15. Utilisation selon au moins l'une quelconque des revendications 13 à 14, **caractérisée en ce que** les esters contiennent (a) 10-25% en poids de monoesters, (b) 25-40% en poids de diesters et (c) 30-45% en poids de triesters et le cas échéant des tétraesters.

16. Utilisation selon la revendication 15, **caractérisée en ce que** les esters contiennent (a) 12-19% en poids de monoesters, (b) 25-35% en poids de diesters, (c) 30-40% en poids de triesters et (d) 6-11% en poids de tétraesters.

17. Utilisation d'un ester selon l'une quelconque des revendications 10 à 11 comme agent conférant une consistance.
